# EUROPEAN PATENT SPECIFICATION

(11) **EP 1 947 457 B1**
(45) Date of publication and mention of the grant of the patent: **13.07.2011**
(21) Application number: 07000857.8
(22) Date of filing: 17.01.2007
(51) Int. Cl.: G01N 33/543, G01N 33/558

(54) **Lateral-flow test device providing improved test quality**
Lateralfluss-Testvorrichtung mit verbesserter Testqualität
Dispositif de test à flux latéral fournissant une qualité de test améliorée

(43) Date of publication of application: 23.07.2008
(73) Proprietor: AraGen Biotechnology Co. Ltd., 11710 Naor (JO)
(72) Inventor: Badwan, Adnan, Dr., 11185 Amman (JO); Murshed Mohammed, Murshed Abdel-Quader, Dr., 11118 Amman (JO)
(74) Representative: Winkler, Andreas Fritz Ernst

(56) References cited:
- NIE HUA-LI ET AL: "Adsorption of papain with Cibacron Blue F3GA carrying chitosan-coated nylon affinity membranes." INTERNATIONAL JOURNAL OF BIOLOGICAL MACROMOLECULES 28 FEB 2007, vol. 40, no. 3, 14 August 2006 (2006-08-14), pages 261-267, XP002426928
- DUBITSKY A: "Blocking strategies for nylon membranes used in enzyme-linked immunosorbent assays" INTERNET ARTICLE, [Online] July 1997 (1997-07), pages 1-4, XP002426929 Retrieved from the Internet: URL:http://www.devicelink.com/ivdt/archive /97/07/012.html> [retrieved on 2007-03-26]
- SCHNEIDER E ET AL: "DETECTION OF AFLATOXINS TRICHOTHECENES OCHRATOXIN A AND ZEARALENONE BY TEST STRIP ENZYME IMMUNOASSAY A RAPID METHOD FOR SCREENING CEREALS FOR MYCOTOXINS" FOOD AND AGRICULTURAL IMMUNOLOGY, vol. 3, no. 3-4, 1991, pages 185-193, XP002933368
- TANAKA R ET AL: "A novel enhancement assay for immunochromatographic test strips using gold nanoparticles." ANALYTICAL AND BIOANALYTICAL CHEMISTRY, vol. 385, no. 8, August 2006 (2006-08), pages 1414-1420, XP002424110
- KIM J-K ET AL: "EFFECTS OF POLYMER GRAFTING ON A GLASS SURFACE FOR PROTEIN CHIP APPLICATIONS" COLLOIDS AND SURFACES B: BIOINTERFACES, vol. 33, no. 2, 2004, pages 67-75, XP009076574

## Description

The present invention refers to a use of a positively charged water-soluble chitosan of molecular weight ≤ 2,000 in the manufacture of a nitrocellulose membrane based lateral-flow test device providing improved quality.

### Background of the Invention

In recent years, the *in vitro* diagnostics (IVD) industry has made enormous efforts to develop immunochromatographic tests. Such tests have found applications in both clinical and non-clinical fields. A clinical utility of this test format has been shown for more than 150 different analytes, and many of them are target now of commercially available diagnostic products.

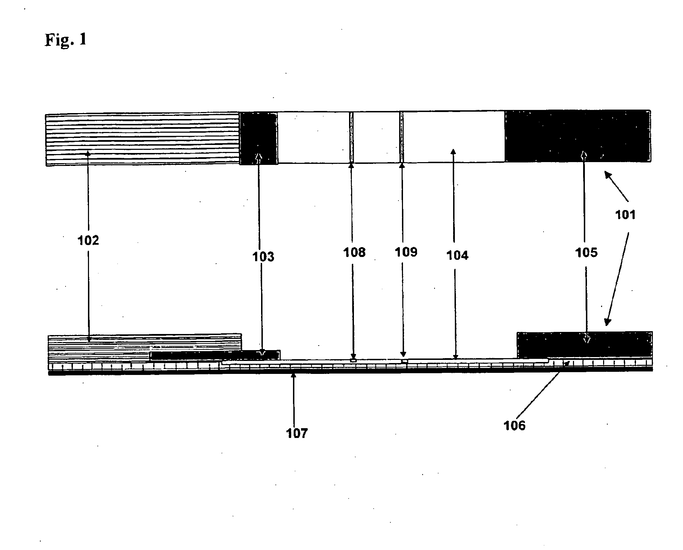

Rapid immunochromatographic test devices, e.g. in the form of a test strip, are made up of a number of components (see Fig. 1). Such a test strip 101 commonly includes a sample pad 102, a conjugate pad 103, a membrane 104, e.g. a nitrocellulose membrane, and an absorbent pad 105. The membrane 104 is usually attached by means of an adhesive 106 to a supporting backing 107, e.g. made of plastic. In practice, the user dispense a patient sample (usually urine or whole blood) onto the sample pad 102. The sample then flows through the sample pad 102 into the conjugate pad 103, where it mixes with and releases the detector reagent. This mixture then flows across the membrane 104, where it binds with the test and control reagents located in the capture test zone 108 (sample zone) and negative control zone 109, respectively. When the mixture binds to the reagent that forms the test line, a positive result is indicated. The colour intensity of the test line is proportional to the concentration of analyte in the sample. Excess sample that flows beyond the test and control zones 108, 109 is taken up in the absorbent pad 105.

Rapid immunochromatographic test devices for diagnostic purposes are easy to operate and thus do not only contribute to the comfort of professional users, e.g. medical stuff, but also allow the operation by non-professionals users, e.g. most patients.

One serious problem associated with the manufacturing of membrane-based lateral-flow test devices are the costs of the in-process quality control during nitrocellulose membrane printing. The manufacturer is forced to test a part from each end of each antibody/antigen printed card to ensure the continuity of the printed lines. A huge volume of expensive biological controls should be used to test these parts. In spite of huge expenditure, this quality control procedure is not efficient because cuts and irregularity of lines within the same card are not subject of testing. Thus, any bubbles in the sprayed solution and/or machine errors could yield discontinuous lines. As a consequence, false negative results may be produced.

Casein, non-fat dry milk and other proteins are used as blocking agents due to their ability to block the negative charged surface of nitrocellulose membranes except the already occupied parts (protein-printed). Blocking of the negative charged surface of nitrocellulose membranes by these proteins is due to its positively charged amino acids within these proteins.

H.-L. Nie and L.-M. Zhu (2007, available online 14 August 2006) (Adsorption of papain with Cibacron Blue F3GA carrying chitosan-coated nylon affinity membranes. Int. J. Biol. Macromol. 40: 261-267) discloses a covalent coupling of chitosan to an activated nylon membranes.

A. Dubitsky (1997) (Blocking strategies for nylon membranes used in enzyme-linked immunosorbent assays. IVD Technology Magazine, internet article, pages 1-4) discloses that the most effective blocking formulation for all membrane types including nylon membranes is a solution of casein.

E. Schneider et al. (1991) (Detection of aflatoxins, trichothecenes, ochratoxin A and zearalenone by test strip enzyme immunoassay: a rapid method for screening cereals for mycotoxins. Food & Agriculture Immunology 3: 185-193) discloses the blocking of Immunodyne Immunoaffinity membrane by fetal calf serum in phosphate- buffered saline.

R. Tanaka et al. (2006) (A novel enhancement assay for immunochromatographic test strips using gold nanoparticles. Anal Bioanal Chem 385: 1414-1420) discloses the preparation of an immunochromatographic test strip comprising blocking of a nitrocellulose membrane by casein in order to prevent nonspecific adsorption.

J.K. Kim et al. (2004) (Effects of polymer grafting on a glass surface for protein chip applications. Colloids Surf B 33: 67-75) discloses a modification of glass surfaces by hydrophilic polymers using e.g. chitosan.

It would be of advantage to add a new feature in the manufacturing of the membrane based lateral-flow test devices to improve the quality of membrane printing processes during and after the manufacturing of these products.

It is an object of the present invention to provide improved quality lateral-flow test devices.

### Summary of the Invention

The object of the present invention is solved by a use of at least one positively charged water-soluble chitosan of molecular weight ≤ 2,000 as a membrane-blocking agent in the manufacture of a rapid immunochromatographic test device.

The object of the present invention is further solved by a method for manufacturing a rapid immunochromatographic test device comprising a step of applying at least one positively charged water-soluble chitosan of molecular weight ≤ 2,000 to a negatively charged synthetic membrane.

In one embodiment, the membrane comprises nitrocellulose, and preferably consists of nitrocellulose.

In one embodiment, the step of applying comprises a spraying of a solution of the positively charged water-soluble chitosan onto the membrane.

In an alternative embodiment, the step of applying comprises a spotting of a solution of the positively charged water-soluble chitosan, preferably of the positively charged water-soluble chitosan onto one end of the membrane.

In another alternative embodiment, the step of applying comprises a dipping of one end of the membrane into a solution of the positively charged water-soluble chitosan.

The object of the present invention is further solved by a rapid immunochromatographic test device comprising a negatively charged synthetic membrane blocked by positively charged water-soluble chitosan of molecular weight ≤ 2,000..

The object of the present invention is further solved by a use of the rapid immunochromatographic test device according to the present invention for a detection of an analyte selected from hCG, *H. pylori* antibody and HIV-1 or HIV-2 antibody in a sample, preferably urine, obtained from a subject, preferably human.

Surprisingly, we recognized that positively charged water-soluble low molecular weight chitosan oligosaccharides (molecular weight ≤ 2,000), containing amino groups can function as a membrane-blocking agent due to their ability to block the negative charged surface of nitrocellulose membranes except the already occupied parts (protein-printed).

By blocking the nitrocellulose membrane using a blocking solution comprising chitosan oligosaccharides, printed lines appear as white lines on a yellowish background and allow for in-process control of reagents printing on nitrocellulose membrane. Other negatively charged synthetic membranes are subject of the same application.

Chitosan application could be done by spraying an appropriate solution onto the entire membrane surface or by applying the solution to one end of the membrane which then flows to the other end through capillary flow. Chitosan oligosaccharides have a yellowish colour that changes the colour of the membrane from white to yellow. Intensity of yellow colour depends on the concentration of used chitosan solution.

### Detailed Description of the Invention

### Brief Description of the Figures

- Figure 1: shows top and side views of a typical rapid-flow immunochromatographic test device in the form of a test strip 101 including a sample pad 102, a conjugate pad 103, a membrane 104 that incorporates capture reagents, an absorbent pad 105, an adhesive 106, a supporting backing 107, a test zone 108, and a control zone 109.
- Figure 2: shows a top view of a printed nitrocellulose membrane during the manufacturing of a rapid-flow immunochromatographic test device.
- Figure 3: shows a top view of a printed nitrocellulose membrane after the application of chitosan solution during the manufacturing of a rapid-flow immunochromatographic test device.
- Figure 4: shows top and side views of a rapid-flow immunochromatographic test device treated with chitosan, e.g. before sample application.
- Figure 5: shows top and side views of a rapid-flow immunochromatographic test device treated with chitosan after sample application, i.e. after sample flow and interactions within the device.
- Figure 6: shows top and side views of a rapid-flow immunochromatographic test device treated with chitosan that is ready for read.
- Figure 7: shows top and side views of a rapid-flow immunochromatographic test device treated with chitosan for pregnancy (hCG hormone) detection.
- Figure 8: shows top and side views of a rapid-flow immunochromatographic test device treated with chitosan for *H. pylori* antibody detection.
- Figure 9: shows top and side views of a rapid-flow immunochromatographic test device treated with chitosan for HIV antibody detection.

### EXAMPLES

### EXAMPLE 1: Quality control

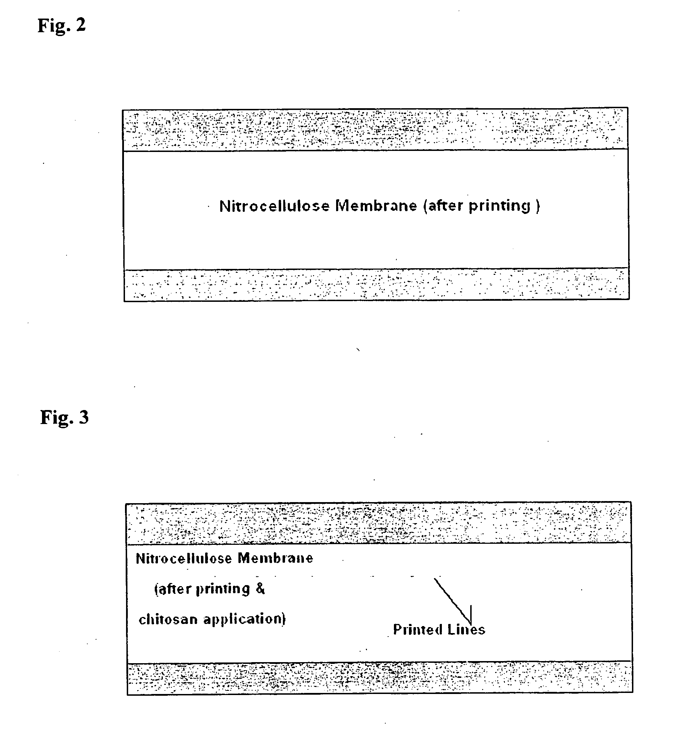

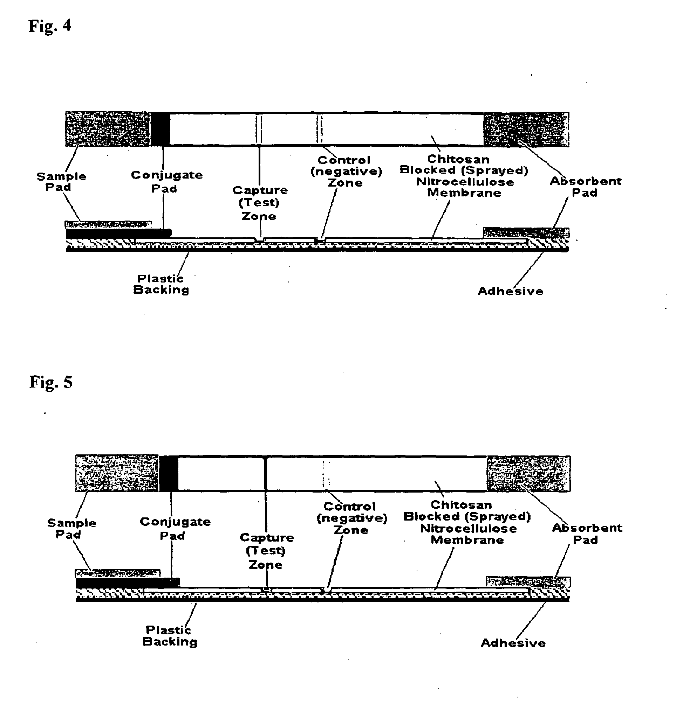

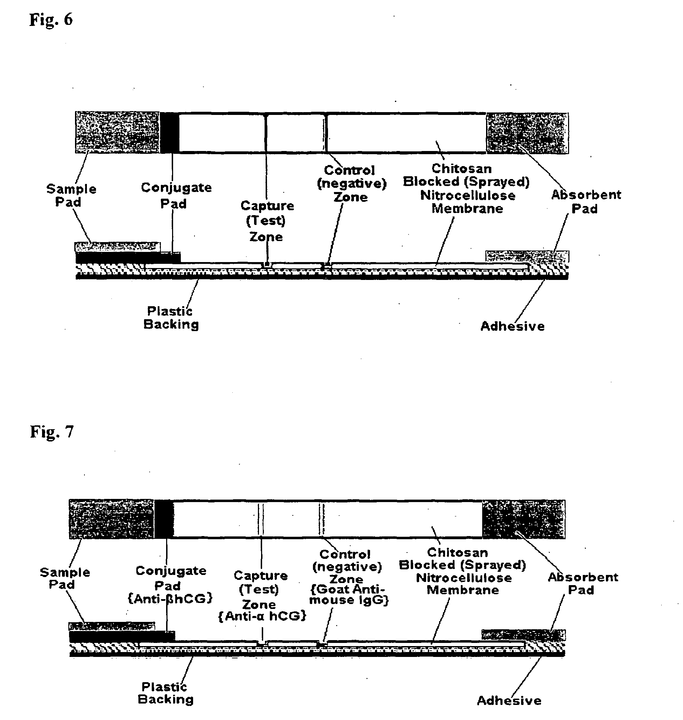

In one embodiment, the invention serves for quality control of protein printing onto nitrocellulose membrane during the manufacturing of lateral-flow test devices of the present invention using chitosan oligosaccharide solution. Printed lines could not be visually distinguished before the application of chitosan oligosaccharide solution (Fig. 2). Membrane blocking using chitosan oligosaccharide solution will distinguish these lines (Fig. 3-5). A solution of chitosan oligosaccharides (that contains amino groups as active groups) blocks all active groups (nitro group) on the surface of nitrocellulose membrane except the protein-printed sites (lines). Coloured membrane allows detecting cuts within the protein-printed lines. Upon sample (fluid) application onto the sample pad, the fluid passes through the nitrocellulose membrane toward the absorbent pad, and an interaction occur between the analyte (binding to the conjugate) within the sample and the printed protein (antibodies/antigens). A red coloured line appears onto a coloured background (chitosan oligosaccharide coloured nitrocellulose membrane) indicating the result is positive or negative depending on the appearance or not of the test line (Fig. 6).

### EXAMPLE 2: hCH antigen detection test strip

A rapid immunographic test device for the detection of hCG is shown in Fig. 7. For the manufacture, 0.3% chitosan oligosaccharide (molecular weight ≤ 2,000) solution was spayed onto the nitrocellulose membrane. The resultant membrane is yellowish and the printed proteins or cuts are distinguishable. After application of a positive sample, the printed lines appear.

### EXAMPLE 3: H. pylori antigen detection test strip

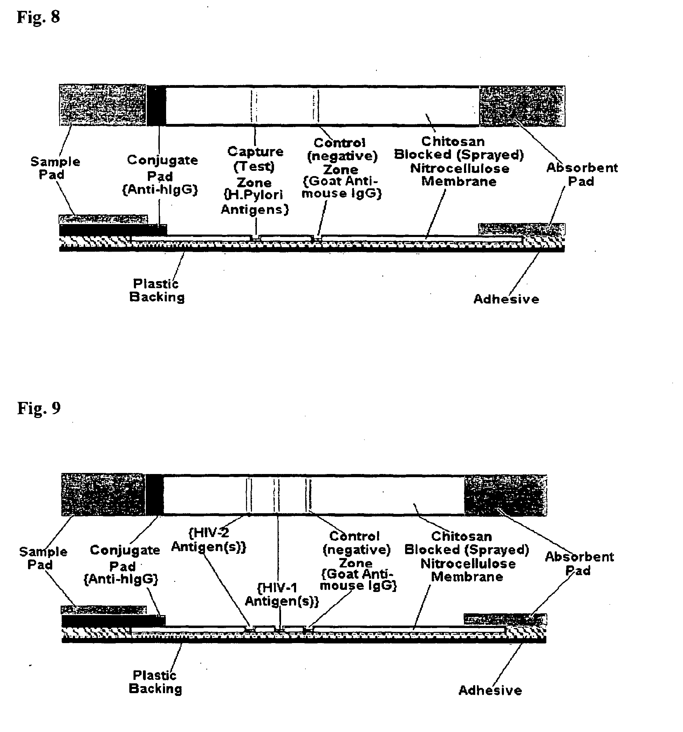

A rapid immunographic test device for the detection of *H. pylori* antigen is shown in Fig. 8. For the manufacture, 0.3% chitosan oligosaccharide (molecular weight ≤ 2,000) solution was applied to one end of a nitrocellulose membrane, i.e. the sample application side. The resultant membrane is yellowish and the printed proteins or cuts are distinguishable. After application of a positive sample, the printed lines appear.

### EXAMPLE 4: HIV-1/2 antibody detection test strip

A rapid immunographic test device for the detection of HIV-1/2 antibody is shown in Fig. 9. For the manufacture, 0.3% chitosan oligosaccharide (molecular weight ≤ 2,000) solution was applied to one end of a nitrocellulose membrane, i.e. the sample application side. The resultant membrane is yellowish and the printed proteins or cuts are distinguishable. After application of a positive sample the three printed lines appear.

## Claims

1. A use of at least one positively charged water-soluble chitosan of molecular weight ≤ 2,000 for blocking a negatively charged synthetic membrane in the manufacture of a rapid immunochromatographic test device.

2. A method for manufacturing a rapid immunochromatographic test device comprising a step of applying at least one positively charged water-soluble chitosan of molecular weight ≤ 2,000 to a negatively charged synthetic membrane.

3. The method according to claim 2, wherein the membrane comprises nitrocellulose.

4. The method according to claim 2 or 3, wherein the step of applying comprises a spraying of a solution of the positively charged water-soluble chitosan onto the membrane.

5. The method according to claim 2 or 3, wherein the step of applying comprises a spotting of a solution of the positively charged water-soluble chitosan onto one end of the membrane.

6. The method according to claim 2 or 3, wherein the step of applying comprises a dipping of one end of the membrane into a solution of the positively charged water-soluble chitosan.

7. A rapid immunochromatographic test device comprising a negatively charged synthetic membrane blocked by positively charged water-soluble chitosan of molecular weight ≤ 2,000.

8. Use of the rapid immunochromatographic test device according to claim 7 for a detection of an analyte selected from human chorionic gonadotropin (hCG), *H. pylori* antibody and HIV-1 or HIV-2 antibody in a sample, preferably urine, obtained from a subject, preferably human.

## Patentansprüche

1. Verwendung wenigstens eines positiv geladenen wasserlöslichen Chitosans mit einem Molekulargewicht von ≤ 2.000 zum Blockieren einer negativ geladenen synthetischen Membran bei der Herstellung einer immunochromatographischen Schnelltestvorrichtung.

2. Verfahren zur Herstellung einer immunochromatographischen Schnelltestvorrichtung, welches einen Schritt umfasst, dass wenigstens ein positiv geladenes wasserlösliches Chitosan mit einem Molekulargewicht ≤ 2.000 auf eine negativ geladene synthetische Membran aufgebracht wird.

3. Verfahren nach Anspruch 2, wobei die Membran Nitrocellulose umfasst.

4. Verfahren nach Anspruch 2 oder 3, wobei der Schritt des Aufbringens ein Sprühen einer Lösung des positiv geladenen wasserlöslichen Chitosans auf die Membran umfasst.

5. Verfahren nach Anspruch 2 oder 3, wobei der Schritt des Aufbringens ein Auftüpfeln einer Lösung des positiv geladenen wasserlöslichen Chitosans auf ein Ende der Membran umfasst.

6. Verfahren nach Anspruch 2 oder 3, wobei der Schritt des Aufbringes ein Eintauchen eines Endes der Membran in eine Lösung des positiv geladenen wasserlöslichen Chitosans umfasst.

7. Immunochromatographische Schnelltestvorrichtung, die eine negativ geladene synthetische Membran umfasst, die durch positiv geladenes wasserlösliches Chitosan mit einem Molekulargewicht ≤ 2.000 blockiert ist.

8. Verwendung der immunochromatographischen Schnelltestvorrichtung nach Anspruch 7 zum Nachweis eines Analyten, der ausgewählt ist aus menschlichem Choriongonadotropin (hCG), *H*. *pylori*-Antikörper und HIV-1- oder HIV-2-Antikörper, in einer Probe, vorzugsweise Urin, die von einem Patienten, vorzugsweise einem Menschen, erhalten ist.

## Revendications

1. Utilisation d'au moins un chitosane soluble dans l'eau à charge positive présentant un poids moléculaire ≤ 2,000 destiné à bloquer une membrane synthétique à charge négative lors de la fabrication d'un dispositif de test immunochromatographique rapide.

2. Procédé de fabrication d'un dispositif de test immunochromatographique rapide comprenant une étape qui consiste à appliquer au moins un chitosane soluble dans l'eau à charge positive présentant un poids moléculaire ≤ 2,000 à une membrane synthétique à charge négative.

3. Procédé selon la revendication 2, dans lequel la membrane comprend la nitrocellulose.

4. Procédé selon la revendication 2 ou 3, dans lequel l'étape d'application comprend la pulvérisation d'une solution du chitosane soluble dans l'eau à charge positive sur la membrane.

5. Procédé selon la revendication 2 ou 3, dans lequel l'étape d'application comprend l'application par touches d'une solution du chitosane soluble dans l'eau à charge positive sur une extrémité de la membrane.

6. Procédé selon la revendication 2 ou 3, dans lequel l'étape d'application comprend un trempage d'une extrémité de la membrane dans une solution du chitosane soluble dans l'eau à charge positive.

7. Dispositif de test immunochromatographique rapide comprenant une membrane synthétique à charge négative bloquée par un chitosane soluble dans l'eau à charge positive de poids moléculaire ≤ 2,000.

8. Utilisation du dispositif de test immunochromatographique selon la revendication 7 pour une détection d'une substance à analyser choisie parmi la gonadotrophine chorionique humaine (hCG), des anticorps H.pylori et des anticorps VIH-1 ou VIH-2 dans un échantillon, de préférence l'urine, obtenue d'un sujet, de préférence humain.
